# EUROPEAN PATENT APPLICATION

(11) **EP 2 929 868 A1**
(43) Date of publication of application: **14.10.2015**
(21) Application number: 13862861.5
(22) Date of filing: 06.12.2013
(51) Int. Cl.: A61J 3/00, A61J 1/05, G09F 3/00, G09F 3/02

(54) **MEDICAL CONTAINER PACKAGE, AND MEDICAL CONTAINER MANAGEMENT METHOD**

(30) Priority: 10.12.2012 JP 2012269855
(71) Applicant: LINTEC Corporation, Tokyo 173-0001 (JP)
(72) Inventor: MITSUHASHI Masafumi, Tokyo 173-0001 (JP); MURAKAMI Takakazu, Tokyo 173-0001 (JP)
(74) Representative: Hoffmann Eitle
(86) International application number: PCT/JP2013/082760
(87) International publication number: WO 2014/092004

(57) **Abstract**

A medical container package (10) includes: an identification information label (50) indicating donor-identification information (BC) that is to be assigned to each donor of a medical fluid; and a blood bag (20) (a medical container) configured to contain the medical fluid. The medical container has been provided with donor-identification information that is the same in content as the donor-identification information indicated by the identification information medium.

## Description

### TECHNICAL FIELD

The present invention relates to a medical container package and a medical container management method.

### BACKGROUND ART

Typically, when a medical fluid such as blood collected from a donor is contained in a medical container such as a blood bag, the consistency between the donor of the medical fluid and the medical container containing the medical fluid is ensured using identification information media (e.g., labels) with donor-identification information (e.g., a barcode) for identifying each donor. The labels with a barcode are stuck on, for instance, a blood bag, a blood collection tube and a medical record when blood is collected from a donor (i.e., a blood donor) in a hospital, a blood donation vehicle, a blood donation room or the like. The collected blood is subjected to a blood separation process, a blood test or the like in a predetermined hospital or blood center.

For instance, a blood collection container disclosed in Patent Literature 1 includes a blood collection needle, a bag-shaped blood collection container body (a blood bag), blood collection decompression tubes, and one label for the blood collection container body and three labels for the blood collection decompression tubes that are stuck on the blood collection container body. In collecting blood, these labels are filled with necessary information, and the labels for the blood collection decompression tubes are peeled off and stuck on the blood collection decompression tubes. According to Patent Literature 1, the management of the blood collection decompression tubes and the like by sticking the labels can ensure the consistency between a blood donor and blood of the blood donor, which is contained in the blood collection container body and the blood collection decompression tubes, with enhanced reliability.

A blood bag management method disclosed in Patent Literature 2 includes: reading a barcode on a patient card using a barcode reader to obtain patient information; manufacturing a plurality of labels printed with a barcode corresponding to the patient information; and sticking the plurality of labels on a blood bag and a storing container. After blood is collected from a patient and contained in the blood bag, the barcode on the patient card, the barcode on the blood bag and the barcode on the storing container are read to confirm that these barcodes are the same. The storing container is then locked after the blood bag is stored therein. In taking the blood bag out of the storing container, it is confirmed that the barcode on the patient card and the barcode on the storing container are the same, and then the storing container is unlocked. According to Patent Literature 2, the blood bag management method can easily and reliably prevent a wrong blood bag from being taken.

### CITATION LIST

### PATENT LITERATURE(S)

Patent Literature 1: JP-A-2-71728
Patent Literature 2: JP-A-6-343679

### SUMMARY OF THE INVENTION

### PROBLEM(S) TO BE SOLVED BY THE INVENTION

Patent Literature 1 requires the one label for the container body and the three labels for the blood collection decompression tubes to be filled in with necessary information every time when blood is collected. In other words, it is necessary to write the necessary information four times per donor. Due to such an increased workload, the information may be, for instance, miswritten or forgotten to be written.

Patent Literature 2 requires preparation of a plurality of labels, which are to be stuck on the blood bag and the storing container, independent of preparation of the blood bag. The plurality of labels should be stuck one by one every time when blood is collected. Such an increased workload may cause problems such as sticking a wrong label or forgetting to stick a label.

An object of the invention is to provide a medical container package and a medical container management method, capable of reducing a workload for sticking an identification information medium provided with donor-identification information for identifying a donor of a medical fluid to a medical container.

### MEANS FOR SOLVING THE PROBLEM(S)

According to a first aspect of the invention, a medical container package includes: an identification information medium indicating donor-identification information that is to be assigned to each donor of a medical fluid; and a medical container configured to contain the medical fluid, the medical container having been provided with donor-identification information that is the same in content as the donor-identification information indicated by the identification information medium.

According to a second aspect of the invention, a medical container management method includes managing a medical container configured to contain a medical fluid on a basis of each donor of the medical fluid using a medical container package, the medical container package including: the medical container having been provided with donor-identification information that is to be assigned to the each donor, the donor-identification information being generated in advance; and an identification information medium indicating donor-identification information that is the same in content as the donor-identification information provided on the medical container.

According to a third aspect of the invention, a medical container management method includes: providing donor-identification information that is to be assigned to each donor of a medical fluid to a first medical container in advance; preparing an identification information medium printed with donor-identification information that is the same in content as the donor-identification information provided on the first medical container; packing the first medical container in a medical container package; providing the identification information medium to the medical container package; reading the donor-identification information provided on the first medical container; storing the read donor-identification information in relation to information about the each donor; and sticking the identification information medium to a second medical container.

The medical container package of the first aspect includes: an identification information medium indicating donor-identification information; and a medical container having been provided with donor-identification information that is the same in content as the donor-identification information indicated by the identification information medium. The medical container package of the first aspect eliminates the need for a person who collects a medical fluid such as blood (a collecting person) to stick the identification information medium to each medical container every time when he/she collects the medical fluid.

In other words, the medical container package of the first aspect can reduce a workload for sticking the identification information medium to each medical container.

A typical collecting work to be performed by a collecting person is troublesome and requires a large workload due to the necessity of sticking the identification information medium to each medical container every time when he/she collects a medical fluid. Consequently, the collecting person sometimes, for instance, sticks a wrong label or forgets to stick a label, which may result in destroying the consistency between the donor and the medical container.

However, the medical container package of the first aspect reduces a workload for sticking the identification information medium on the medical container, thereby restraining problems such as sticking a wrong identification information medium and forgetting to stick the identification information medium, and thus reducing the possibility of destroying the consistency between the donor and the medical container. The collected medical fluid can thus be managed with improved accuracy.

The medical container management method of the second aspect is implemented using a medical container package, which includes: a medical container having already been stuck with donor-identification information; and an identification information medium, to manage the medical container. The medical container management method eliminates the need for a collecting person to stick the identification information medium to each medical container taken out of the medical container package every time when he/she collects a medical fluid.

In other words, the medical container management method of the second aspect can reduce a workload for sticking the identification information medium to each medical container. This results in restraint of, for instance, sticking the identification information medium on a wrong medical container and forgetting to stick the identification information medium. Therefore, the possibility of destroying the consistency between the donor and the medical container can be reduced to manage the collected medical fluid with improved accuracy.

The medical container management method of the third aspect is implemented using a medical container package, which includes a first medical container having been stuck with donor-identification information that is to be assigned to each donor of a medical fluid. The medical container package also includes an identification information medium printed with the same donor-identification information stuck on the first medical container. A collecting person reads the donor-identification information stuck on the first medical container using a reader or the like. The collecting person then stores the read donor-identification information in, for instance, a storage of a controller in relation to information about the donor. Further, the collecting person sticks the identification information medium to a second medical container.

The medical container management method of the third aspect can reduce a workload for sticking the identification information medium on the medical container. This results in restraint of, for instance, sticking the identification information medium on a wrong medical container and forgetting to stick the identification information medium. Therefore, the possibility of destroying the consistency between the donor and the medical container can be reduced to manage the collected medical fluid with improved accuracy. Further, according to the third aspect, the donor-identification information is stored in relation to the information about the donor, thereby improving the accuracy of management of the collected medical fluid and the traceability thereof.

### BRIEF DESCRIPTION OF DRAWING(S)

Fig. 1 schematically shows an arrangement of a medical container package according to a first exemplary embodiment.
Fig. 2 is a perspective view of the medical container package.
Fig. 3A is a perspective view of a collection container storing case for storing collection containers for containing a medical fluid, for showing the collection container storing case in which none of the collection containers has yet been stored.
Fig. 3B is another perspective view of the collection container storing case for storing the collection containers for containing the medical fluid, for showing a bottom surface of the collection container storing case with identification information media being placed thereon.
Fig. 3C is still another perspective view of the collection container storing case for storing the collection containers for containing the medical fluid, for showing the collection containers arranged side by side in the collection container storing case having the bottom surface on which the identification information media are placed.
Fig. 3D is a further perspective view of the collection container storing case for storing the collection containers for containing the medical fluid, for showing the collection containers arranged side by side and the identification information media placed thereon.
Fig. 4 is a block diagram showing a schematic arrangement of a medical container management system according to the first exemplary embodiment.
Fig. 5 is a perspective view of a medical container package according to a second exemplary embodiment.
Fig. 6 is a schematic sectional view of identification information media according to the second exemplary embodiment stuck on a cover.
Fig. 7A is a plan view of a first united label body according to a third exemplary embodiment stuck on a cover of a medical container package, the first united label body including united identification information media.
Fig. 7B is a schematic sectional view of the first united label body stuck on the cover of the medical container package.
Fig. 8A is a plan view showing a part of a process for sticking each of the identification information media to the collection container.
Fig. 8B is a schematic sectional view showing the part of the process for sticking each of the identification information media to the collection container.
Fig. 9 is a plan view of a second united label body according to a fourth exemplary embodiment.

### DESCRIPTION OF EMBODIMENT(S)

### First Exemplary Embodiment

A first exemplary embodiment of the invention will be described below with reference to the attached drawings.

In the description of the first exemplary embodiment, a medical fluid is, for instance, blood. However, the medical fluid may be different from blood without departing from the scope of the invention.

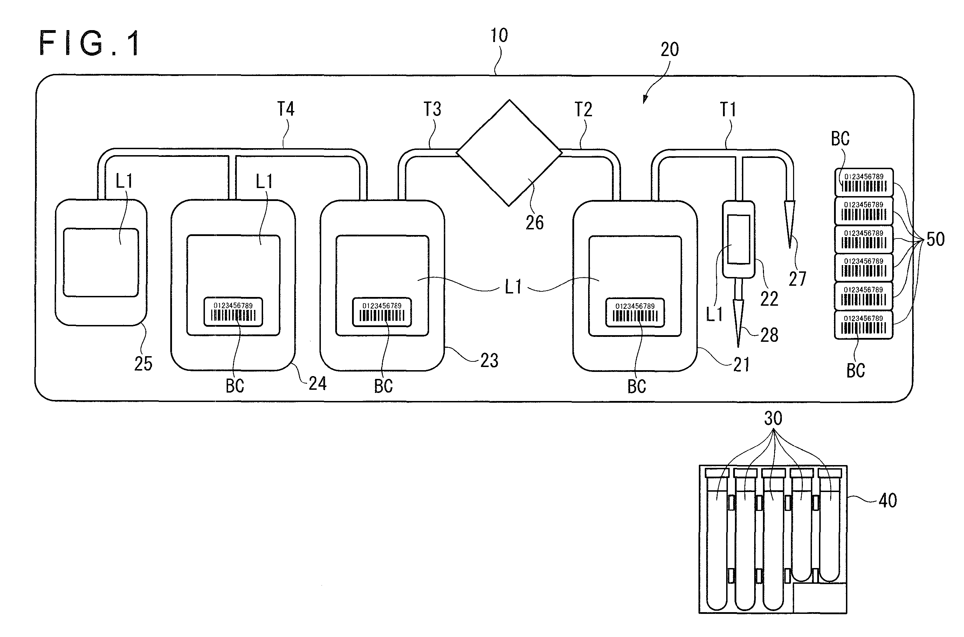

Fig. 1 shows a schematic arrangement of a medical container package 10 according to the first exemplary embodiment. Fig. 1 also shows a schematic arrangement of a blood collection tube case 40 (a collection container storing case) containing a plurality of blood collection tubes 30 (a second medical container) serving as collection containers.

The medical container package 10 includes: a blood bag 20 (a medical container) for containing blood provided by a donor; and identification (ID) information labels 50 (an identification information medium) to be stuck on the blood collection tubes 30. Incidentally, the blood bag 20 serves as a first medical container according to the medical container management method of the invention.

In the first exemplary embodiment, the blood bag 20 includes a first bag 21, a blood test bag 22, a second bag 23, a third bag 24, a fourth bag 25 and a blood filter 26.

The first bag 21 is connected to a blood collection needle 27 disposed upstream thereof through a tube T1. The tube T1 is branched in the middle thereof to be connected to the blood test bag 22. The blood test bag 22 is connected to a needle 28 disposed downstream thereof, the needle 28 being used for supplying blood to the blood collection tubes 30.

The first bag 21 is connected to the blood filter 26 disposed downstream thereof through a tube T2, and the blood filter 26 is connected to the second bag 23 disposed downstream thereof through a tube T3. The second bag 23 is connected to the fourth bag 25 disposed downstream thereof through a tube T4. The tube T4 is branched in the middle thereof to be connected to the third bag 24.

The bags 21, 22, 23, 24, 25 of the blood bag 20 each have an exterior surface stuck with a label L1. The label L1 is printed with information (not shown in Fig. 1) including a trade name of the medical container package 10, a capacity of the blood bag 20, an ingredient of a blood preservative or the like, a manufacturer of the medical container package 10, a production lot number of the medical container package 10.

In the first exemplary embodiment, the label L1 stuck on each of the first bag 21, the second bag 23 and the third bag 24 is also printed with donor-identification information BC to be assigned to each donor. The donor-identification information BC is issued such that each donor is identified by different donor-identification information BC. The label L1 printed with the donor-identification information BC is stuck on each of the first bag 21 and the like to provide the donor-identification information to the blood bag 20 (the medical container).

The donor-identification information BC is preferably in the form of at least one of a barcode and a two-dimensional code. In the first exemplary embodiment, the donor-identification information BC is a barcode.

The donor-identification information BC is preferably printed on the label L1 during the same process where the trade name and the like are printed to simplify a manufacturing process of the blood bag 20. It should be noted that the donor-identification information BC may be in the form of a label and stuck on the label L1 independently of the label L1.

The label L1 includes a printable front sheet and an adhesive layer. The printable front sheet preferably has a printable surface and may be made of any of various sheet materials including: synthetic resin films such as acrylic resin, polycarbonate resin, polyethylene resin, polypropylene resin, polyvinyl chloride resin, acrylonitrile·butadiene·styrene resin, polyester resin (e.g., polyethylene terephthalate) and polyimide resin, and paper materials such as wood-free paper, impregnated paper, glassine paper, coated paper and non-woven cloth. Further, the above sheet materials may be provided with a to-be-printed layer configured to be subjected to a variety of printing processes such as thermal recording, pressure-sensitive printing, thermal transfer recording, laser beam recording and ink-jet recording as needed. Various types of adhesives such as a pressure-sensitive adhesive and a heat-sensitive adhesive are usable for forming the adhesive layer without any specific limitation as long as the adhesive layer can exhibit an adhesive force sufficient to stick on the blood bag. Various materials are usable for forming the adhesive without any specific limitation. For instance, the adhesive may be a rubber, acryl, silicone, urethane or polyester adhesive. Among the above, the acryl or polyester adhesive is suitable because the adhesive force and durability thereof are well-balanced.

The thickness of the sheet material is usually selected in a range from 5 µm to 300 µm, more preferably from 20 µm to 200 µm. The above range is determined in view of the fact that the sheet material with a thickness of less than 5 µm is poor in mechanical strength and thus is likely to deform depending on external pressure conditions. Further, the sheet material with a thickness of more than 300 µm may be difficult to, for instance, roll or cut.

The thickness of the adhesive layer is usually selected in a range from 5 µm to 150 µm, more preferably from 10 µm to 100 µm. The above range is determined in view of the fact that the adhesive layer with a thickness of less than 5 µm may fail to exhibit an adhesive force. Further, the adhesive layer with a thickness of more than 150 µm may make the identification information labels 50 excessively thick as a whole and/or cause a problem such as leakage of the adhesive from sides of the identification information labels 50.

The first bag 21 contains a blood preservative, whereas the fourth bag 25 contains a red-blood-cell preservative. Examples of these preservatives include an acid citrate dextrose-A (ACD-A) solution, a citrate phosphate dextrose (CPD) solution and a mannitol adenine phosphate (MAP) solution.

Examples of a material of the bags 21, 22, 23, 24, 25 of the blood bag 20 include polyolefin resins such as polypropylene and polyethylene, and flexible synthetic resins such as a polyvinyl chloride resin. It should be noted that the material of the bags 21, 22, 23, 24, 25 may be different from the above examples as long as no impurity elutes from the bags into the medical fluid contained therein.

The bags 21, 22, 23, 24, 25 may be provided by putting two polyolefin resin sheet materials on each other and fusing the sheet materials at the respective peripheries thereof into a pouch. The pouch-shaped bag is provided with a blood inlet and a blood outlet at the upside thereof and connected to another bag and/or blood filter through the tubes. The pouch-shaped bag defines therein a containing space having a capacity sufficient for containing a volume of blood collected for one time and an amount of the preservative for the collected blood. In the first exemplary embodiment, the first bag 21, the second bag 23 and the third bag 24 are each capable of containing 400 ml, the fourth bag 25 is capable of containing 150 ml, and the blood test bag 22 is capable of containing 25 ml. It should be noted that the respective capacities of the bags 21, 22, 23, 24, 25 may be different from those of the first exemplary embodiment.

In the first exemplary embodiment, the blood filter 26 is a leukocyte removal filter. The leukocyte removal filter is connected between the first bag 21 and the second bag 23 as described above to remove leukocytes from collected blood before blood products are produced from the blood (prestorage leukocyte reduction). For instance, leukocytes may be removed by hanging the bags with the first bag 21 being situated at an upper side so that the blood gravitationally drops into the tube T2 and passes through the blood filter 26. Removal of leukocytes leads to reduction of occurrence of side effects of blood transduction (e.g., fever) resulting from mismatch in leukocyte type. It should be noted that the blood filter 26 is in a rectangular shape in Fig. 1, but the shape of the blood filter 26 is not specifically limited. For instance, the blood filter 26 may be in a circular shape, an oblong shape, a polygonal shape or an indefinite shape.

The identification information media are preferably adhesive labels. In the first exemplary embodiment, the identification information labels 50 are adhesive labels printed with donor-identification information BC (barcode) that is the same in content as the donor-identification information BC printed on the label L1. The identification information labels 50 each include a printable front sheet and an adhesive layer similarly to the label L1, and further include a release liner protecting the adhesive layer until the adhesive layer is stuck on corresponding one of the blood collection tubes 30 or a medical record. A material of the printable front sheet and a material of the adhesive layer may be appropriately selected from the substances listed above as the material of the label L1. Examples of the release liner include papers such as a polyethylene laminated paper, coated paper and glassine paper, and synthetic resin films such as polyethylene, polypropylene and polyethylene terephthalate. A release agent in a release agent layer of the release liner may be, for instance, a silicone resin, a fluorine resin or a long-chain alkyl resin.

In the case of using a synthetic resin film as the release liner base, the thickness of the release liner is usually approximately in a range from 5 µm to 300 µm, preferably approximately in a range from 20 µm to 200 µm. In the case of using a paper base as the release liner base, the basis weight is usually approximately in a range from 20 g/m² to 450 g/m², preferably approximately in a range from 40 g/m² to 220 g/m². The above ranges are determined in view of the fact that the release liner with a thickness of less than 5 µm in the case of using a synthetic resin film or with a basis weight of less than 20 g/m² in the case of using a paper base is poor in mechanical strength and is likely to deform depending on external pressure conditions. Further, the release linear base with a thickness of more than 300 µm in the case of using a synthetic resin film or with a basis weight of more than 450 g/m² in the case of using a paper base may be difficult to, for instance, roll or cut. The thickness of the release agent layer is, for instance, in a range from 50 nm to 2 µm. In using a synthetic resin film as the release liner base, the thickness of the release agent layer is preferably in a range from 50 nm to 300 nm. In using a paper base as the release liner base, the thickness of the release agent layer is preferably in a range from 300 nm to 2 µm. The above ranges are determined in view of the fact that the release agent layer with a thickness of less than 50 nm in the case of using a synthetic resin film or with a thickness of less than 300 nm in the case of using a paper base may fail to exhibit a release force. Further, the release agent layer with a thickness of more than 300 nm in the case of using a synthetic resin film or with a thickness of more than 2 µm in the case of using a paper base merely requires an excessive amount of material and may increase costs.

Preferably, the identification information labels 50 of the first exemplary embodiment are separably united to one another and includes identification information labels as many as the blood collection tubes 30 and identification information labels as many as medical records that are to be stuck with the identification information. In the first exemplary embodiment, collected blood is partly distributed among five blood collection tubes 30, so that five identification information labels 50 to be stuck on the blood collection tubes 30 and one identification information label 50 to be stuck on a medical record are prepared. The six identification information labels 50 in total are separably united to one another and packed in the medical container package 10 along with the blood bag 20. In the first exemplary embodiment, the six identification information labels 50 are separably united to one another with perforations being provided therebetween. The identification information labels 50 can be separated one by one by tearing the identification information labels 50 along the perforations. The perforations may be replaced with another arrangement for allowing the identification information labels 50 to be separable. For instance, the plurality of identification information labels 50 united to one another may be separated one by one at a cut provided on each of boundaries delimiting the identification information labels 50.

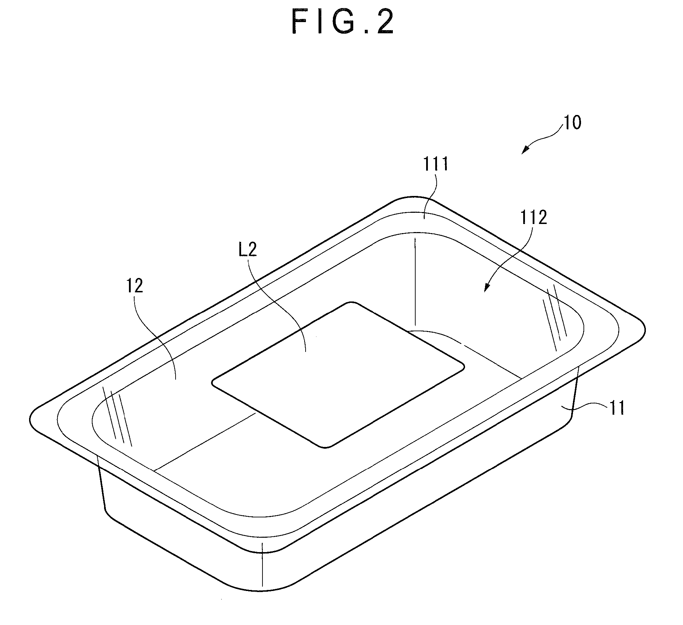

Fig. 2 is a perspective view of the medical container package 10. It should be noted that the blood bag 20 and the identification information labels 50, which are supposed to be contained in the unsealed medical container package 10, are not shown in Fig. 2. The arrangement of the bags of the blood bag 20 packed in the medical container package 10 is not specifically limited. For instance, the bags may be arranged side by side as shown in Fig. 1 or may be arranged in a folded state.

The medical container package 10 includes the blood bag 20, the identification information labels 50, a container body 11 having an opening 112 and containing the blood bag 20 and the identification information labels 50, and a cover 12 covering the opening 112 of the container body 11.

The container body 11, which is in the shape of a substantially rectangular tray with a predetermined depth, has the substantially rectangular opening 112, the peripheral edge of which is provided with a flange 111. Examples of a material of the container body 11 include olefin resins such as polypropylene and polyethylene, polyester resins such as polystyrene resin and polyethylene terephthalate (PET), and blend materials thereof. The container body 11 may include a tray-shaped multilayer body of a plurality of laminated layers or may further include a layer with gas barrier properties. It should be noted that the shape of the container body 11 is not specifically limited. For instance, the container body 11 may be in the shape of a pouch or a box instead of being in the shape of a tray as in the first exemplary embodiment. Further, the size of the container body 11 is also not specifically limited as long as the container body 11 can contain at least the blood bag 20 and the identification information labels 50.

The cover 12 is heat-sealed to the flange 111 to close the opening 112 and seal the container body 11. A label L2 is stuck on the cover 12. The label L2 is printed with information about the medical container package including trade name, capacity, ingredient, manufacturer, production lot number and the like (not shown). The label L2 may further be printed with donor-identification information BC (barcode) that is the same in content as the donor-identification information BC printed on the label L1. The label L2 may be appropriately selected from the substances listed above as the material of the label L1.

In the first exemplary embodiment, the cover 12 is made of a material heat-sealable to the flange 111 of the container body 11. The cover 12 may have a multilayer structure including a sealant layer facing the flange 111 and a base layer. The sealant layer is preferably made of a material heat-sealable to the flange 111, and resins such as polypropylene and polyethylene are usable. The base layer may be made of polyethylene terephthalate, a mixed resin of polypropylene and polyethylene, or a synthetic resin film prepared with nylon, etc. The cover 12 may further include a layer with gas barrier properties.

In the first exemplary embodiment, the blood bag 20 and the like are sealed in the container body 11 by placing the blood bag 20 and the identification information labels 50 in the container body, putting the cover 12, the outline of which is larger than that of the flange 111, on the flange 111, and heat-sealing the flange 111 and the cover 12 to each other.

The outline of the cover 12 is preferably designed to be larger than that of the flange 111 of the container body 11. In order to take out the blood bag 20, the container body 11 is unsealed by pinching a part of the cover 12 outside a corner of the flange 111 and ripping the cover 12 away from the container body 11.

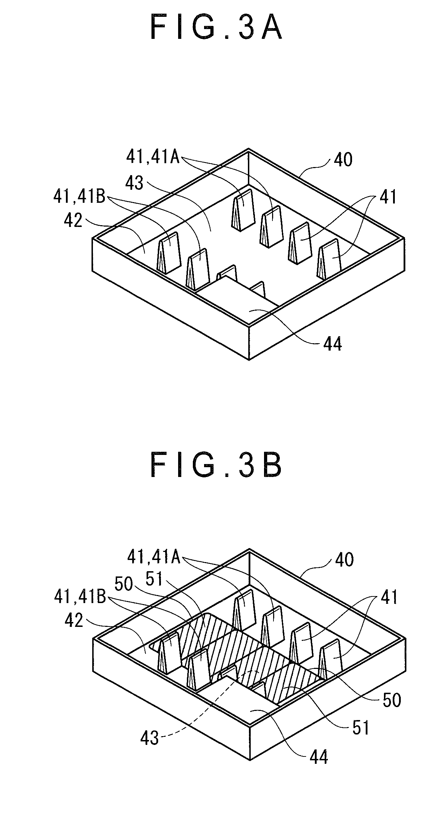

Each of Figs. 3A to 3D is a perspective view of the blood collection tube case 40 capable of containing the plurality of blood collection tubes 30. A method of sticking the identification information labels 50 to the blood collection tubes 30 according to the first exemplary embodiment may include a process described with reference to Figs. 3A to 3D.

Fig. 3A is a perspective view of the empty blood collection tube case 40, in which none of the blood collection tubes 30 has been contained. The blood collection tube case 40 is in the shape of a rectangular parallelepiped box and opens upward. The blood collection tube case 40 includes therein a plurality of partitions 41. In the first exemplary embodiment, the partitions 41 are projections rising from a case bottom 42 of the blood collection tube case 40 and serve to arrange the plurality of blood collection tubes 30 side by side with the longitudinal directions thereof being oriented in the same direction.

In the first exemplary embodiment, eight partitions 41 are arranged in two tiers and four rows. The partitions 41 in each row separate adjacent ones of the blood collection tubes 30 from each other, whereas the partitions in two tiers, namely partitions 41 A, 41B, hold each of the blood collection tubes 30 at two spots in the longitudinal direction, thereby preventing the blood collection tubes 30 from being arranged with inclination in the blood collection tube case 40. The partitions 41A in one tier and the partitions 41B in the other tier are spaced at a predetermined distance, and a portion of the case bottom 42A between the partitions 41A, 41B defines a label mount 43 on which the identification information labels 50 are to be placed. In the first exemplary embodiment, the label mount 43 has a width sufficient to place five of the identification information labels 50 separably united into one piece. It should be noted that the six identification information labels 50, which are separably united to one another when packed in the container body 11, are separated into one identification information label to be stuck on a medical record and five united identification information labels before placed on the label mount 43.

A material of the blood collection tube case 40 may be a synthetic resin, rubber, glass, paper material or the like. The blood collection tube case 40 is preferably made of a light-transmissive material, more preferably a transparent and colorless material. For instance, the blood collection tube case 40 is preferably made of a transparent plastic material.

Fig. 3B is a perspective view of the blood collection tube case 40, in which the united identification information labels 50 are placed on the label mount 43 with an adhesive surface of the adhesive layer 51 of each of the identification information labels 50 facing upward. In order to stick each of the identification information labels 50 to corresponding one of the blood collection tubes 30 at a predetermined position, a dimension of a space between the partitions 41A and the partitions 41B is preferably substantially the same as a dimension of an outline of a united body of the plurality of identification information labels 50. With the above arrangement, as long as the united body of the plurality of identification information labels 50 is placed on the label mount 43, the eight partitions 41 serve to prevent the united body from being displaced from a predetermined position where each of the identification information labels 50 can be stuck on corresponding one of the blood collection tubes 30.

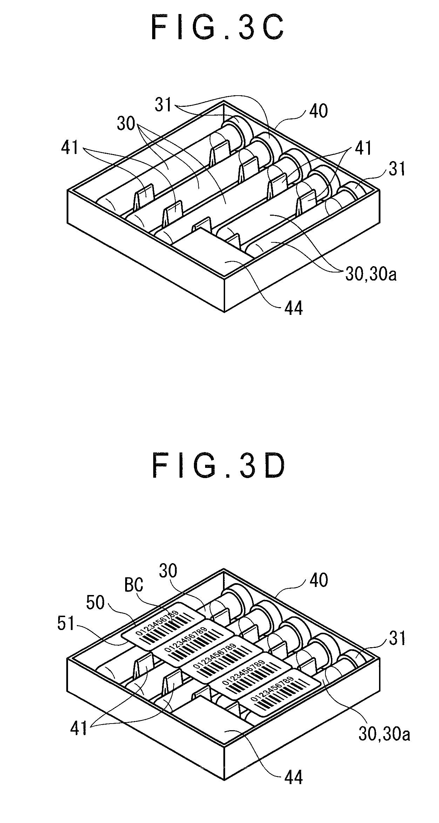

Fig. 3C is a perspective view of the blood collection tube case 40, in which the blood collection tubes 30 are arranged side by side on the identification information labels 50. As shown in Fig. 3C, the plurality of blood collection tubes 30 are separated from one another by the partitions 41 with the respective longitudinal directions of the blood collection tubes 30 being oriented in the same direction. Each of the identification information labels 50 is stuck on corresponding one of the blood collection tubes 30 by placing the blood collection tubes 30 on the identification information labels 50 placed with the adhesive surface of the adhesive layer 51 of each of the identification information labels 50 facing upward. In the first exemplary embodiment, a spacer 44 in the shape of a rectangular parallelepiped is provided at a corner inside the blood collection tube case 40. The spacer 44 serves to not only restrain a blood collection tube 30a, the length of which is shorter than a longitudinal length of the blood collection tube case 40, from being longitudinally displaced inside the blood collection tube case 40, but also prevent the identification information labels 50 from being stuck at a displaced position. Further, the identification information labels 50, which are placed on the label mount 43 as described above, are prevented from being displaced. As a result, each of the identification information labels 50 can be stuck on corresponding one of the blood collection tubes 30 at the predetermined position with accuracy.

The blood collection tubes 30 are each configured to contain a part of blood (a medical fluid). In the first exemplary embodiment, the first blood collection tubes 30 are each in a cylindrical shape and have a first end and a second end in the longitudinal direction, the first end being rounded and closed, the second end having an opening to be closed by a plug 31. A material of the blood collection tubes 30 may be a synthetic resin, glass or the like. The blood collection tubes 30 are preferably made of a light-transmissive material, more preferably a transparent and colorless material. The plug 31 may be a rubber plug, a seal plug, a plastic plug or the like. It the first exemplary embodiment, the five blood collection tubes 30 are attached with the plugs 31, which are preferably differently colored so that intended use purposes of the blood in the blood collection tubes 30 can be easily understood. In the first exemplary embodiment, the blood collection tubes 30 are each in a cylindrical shape in terms of mechanical strength, but may be in a polygonal shape or the like without any specific limitation.

Fig. 3D is a perspective view of the blood collection tube case 40, in which the blood collection tubes 30 are arranged side by side before the identification information labels 50 are placed on the label mount 43, and then the identification information labels 50 are placed on the blood collection tubes 30 with the adhesive surface of the adhesive layer 51 of each of the identification information labels 50 facing downward. In other words, a process for sticking described with reference to Fig. 3D is different from the process described with reference to Figs. 3B and 3C.

As shown in Fig. 3D, the plurality of blood collection tubes 30 are separated from one another by the partitions 41 with the respective longitudinal directions of the blood collection tubes 30 being oriented in the same direction. The united identification information labels 50 are placed on the blood collection tubes 30 with the adhesive surface of the adhesive layer 51 of each of the identification information labels 50 being facing downward, thereby sticking each of the identification information labels 50 to corresponding one of the blood collection tubes 30. It should be noted that the height of the partitions 41 (a dimension from the case bottom 42 to an upper end of each of the partitions 41) is larger than the diameter of the blood collection tubes 30 in the first exemplary embodiment. Further, the dimension of the space between the partitions 41A and the partitions 41B is substantially the same as the dimension of the outline of the united body of the plurality of identification information labels 50. With this arrangement, when the blood collection tubes 30 are placed in the blood collection tube case 40, the upper end of each of the partitions 41 projecting above the blood collection tubes 30 determines a position where the identification information labels 50 are to be placed. As a result, each of the identification each of information labels 50 can be stuck on corresponding one of the blood collection tubes 30 at the predetermined position with accuracy.

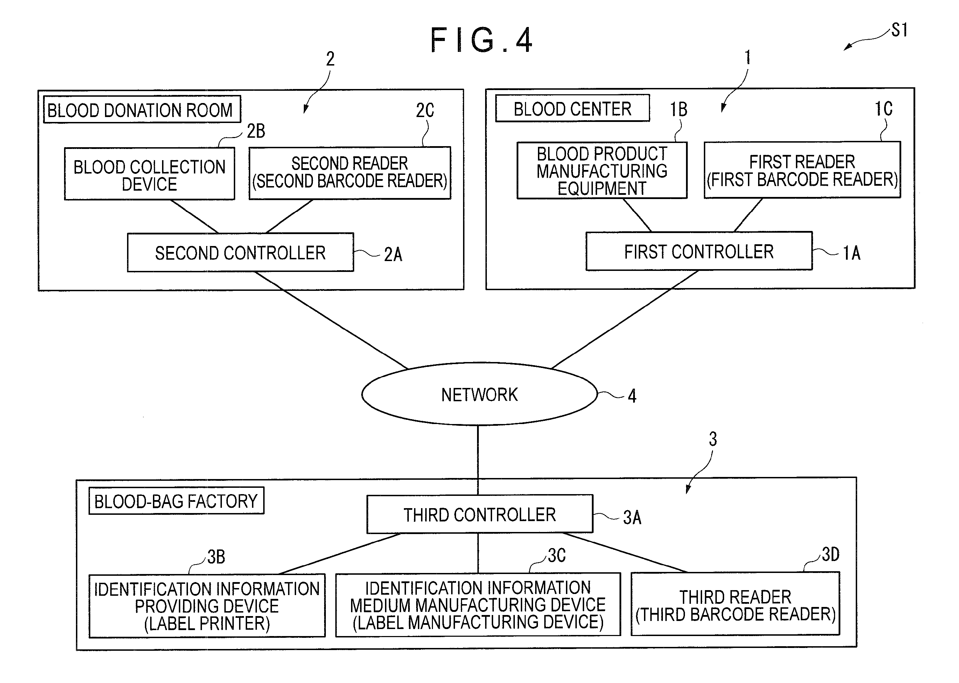

Fig. 4 is a block diagram showing a schematic arrangement of a medical container management system S1 according to the first exemplary embodiment.

The medical container management system of the first exemplary embodiment is configured to manage the blood bag 20 and the blood collection tubes 30 (medical containers). It should be noted that description will be made on an instance where the blood collection tubes 30 (collection containers) are also included in the medical container.

The medical container management system S1 includes a first management system 1 located at a blood center, a second management system 2 located at a blood donation room, and a third management system 3 located at a blood-bag factory. These management systems 1, 2, 3 are configured to intercommunicate through a network 4.

The first management system 1 includes: a first controller 1A that generates the donor-identification information BC to be assigned to each donor who provides his or her blood; blood product manufacturing equipment 1B that manufactures a blood product from collected blood; and a first barcode reader 1C (a first reader).

The first controller 1A is configured to generate unique donor-identification information BC to prevent the same identification information from being assigned to different donors. The blood bag 20 and the identification information labels 50 packed in the same medical container package 10 are provided with the common donor-identification information BC, which is different from donor-identification information provided to a blood bag and identification information labels packed in another medical container package. The first controller 1A transmits the generated donor-identification information BC to a second controller 2A and a third controller 3A through the network 4.

The first controller 1A, which is a computer including an arithmetic unit such as a central processing unit (CPU) and a storage such as a random access memory (RAM) or a read only memory (ROM), may also include an input device for inputting various information to the first controller 1A. The first controller 1A is connected to the blood product manufacturing equipment 1B and the first barcode reader 1C at least by wire or wirelessly to control the blood product manufacturing equipment 1B and the first barcode reader 1C. The first controller 1A is also configured to communicate with the second controller 2A of the second management system 2 and the third controller 3A of the third management system through the network 4. The controllers 1A, 2A, 3A are configured to relate information transmitted/received thereamong to the donor-identification information BC as necessary.

The blood product manufacturing equipment 1B, for instance, removes leukocytes through the blood filter 26 from blood contained in the first bag 21 at a blood donation room (described later), and subjects the second bag 23, which contains the blood from which the leukocytes are removed, to centrifugation to separate red blood cells from blood plasma. The blood product manufacturing equipment 1B may consist of a single device or a plurality of devices.

The blood product manufacturing equipment 1B transmits conditions for manufacturing a blood product and information about the manufactured blood product (blood product manufacturing information) to the first controller 1A. The first controller 1A stores the received blood product manufacturing information in the storage thereof in relation to the donor-identification information BC.

The first barcode reader 1C reads the donor-identification information BC or a barcode printed on the labels L1 on the blood bag 20 and the identification information labels 50 on the blood collection tubes 30, and transmits the read information to the first controller 1A. Based on the information received from the first barcode reader 1C, the first controller 1A performs a predetermined process for relating the received information, for instance, to blood-collecting date and blood-collecting information. The first barcode reader 1C can also read the donor-identification information BC from the identification information labels 50 on the plurality of blood collection tubes 30 placed in the blood collection tube case 40. Specifically, when the identification information labels 50 are placed on the label mount 43 with the adhesive surface of each of the identification information labels 50 facing upward, and at least the case bottom 42 of the blood collection tube case 40 is made of a light-transmissive material, the donor-identification information BC can be read using the first barcode reader 1C through the case bottom 42. Further, when the blood collection tubes 30 are made of a light-transmissive material, it can be seen whether or not a predetermined amount of blood is collected in each of the blood collection tubes 30.

As long as the first barcode reader 1C can read a barcode, the first barcode reader I C is not specifically limited. For instance, a laser-scanning type, a line-sensor type or an area-sensor type may be used.

The second management system 2 includes the second controller 2A, a blood collection device 2B collecting blood from a donor, and a second barcode reader 2C (a second reader).

The second controller 2A is a computer including an arithmetic unit, a storage and an input device in the same manner as the first controller 1A. The second controller 2A is connected to the blood collection device 2B and the second barcode reader 2C at least by wire or wirelessly to control the blood collection device 2B and the second barcode reader 2C. In the first exemplary embodiment, the input device of the second controller 2A is used to input information about the donor. It should be noted that it is not requisite that the first controller 1A generates the donor-identification information BC as described in the first exemplary embodiment. Alternatively, the second controller 2A may generate the donor-identification information BC. In the alternative case, the generated donor-identification information BC is preferably transmitted to the first controller I A and the third controller 3A through the network 4 in advance.

The blood collection device 2B may be, for instance, an automatic blood collection device. The blood bag 20 is set in the automatic blood collection device to automatically collect blood from a donor through the blood collection needle 27. Specifically, the initially collected blood is first contained in the blood test bag 22, whereas the rest of the collected blood is contained in the first bag 21.

The blood collection device 2B transmits the blood-collecting information, such as blood-collecting conditions and the blood pressure of the donor, to the second controller 2A. The second controller 2A stores the received blood-collecting information in the storage thereof in relation to the donor-identification information BC. Further, the second controller 2A may store the blood-collecting information also in relation to the information about the donor having been inputted using the input device as well as the donor-identification information BC.

The second barcode reader 2C reads the donor-identification information BC or a barcode printed on the labels L1 on the blood bag 20, on the label L2 on the medical container package 10, and on the identification information labels 50 on the blood collection tubes 30. The information read by the second barcode reader 2C is transmitted to the second controller 2A. It should be noted that every time when information about the donor is inputted using the input device or the blood-collecting information is inputted, the second controller 2A may store the inputted information in relation to the donor-identification information BC, which is read by the second barcode reader 2C prior to blood collection.

As long as the second barcode reader 2C can read a barcode, the second barcode reader 2C is not specifically limited as well as the first barcode reader 1C.

The third management system 3 includes the third controller 3A, a label printer 3B (an identification information providing device), a label manufacturing device 3C (an identification information medium manufacturing device), and a third barcode reader 3D (a third reader).

The third controller 3A is a computer including an arithmetic unit, a storage and an input device in the same manner as the first controller 1A. The third controller 3A is connected to the label printer 3B, the label manufacturing device 3C and the third barcode reader 3D at least by wire or wirelessly to control the label printer 3B, the label manufacturing device 3C and the third barcode reader 3D. In the first exemplary embodiment, the input device of the third controller 3A is used to input blood bag information (medical container information) about the blood bag 20 including trade name, capacity, ingredient, manufacturer, production lot number and the like. It the first exemplary embodiment, the third controller 3A receives the donor-identification information BC, which is generated by the first controller 1A in advance, through the network 4, and stores the received information in the storage thereof so that the information is ready to be printed on the labels L1, the label L2 and the identification information labels 50. Preferably, the third controller 3A stores the received donor-identification information BC in the storage thereof in advance in relation to the blood bag information such as the production lot number of the blood bag 20. More preferably, the third controller 3A stores the received donor-identification information in the storage thereof in advance in relation to information about the medical container package 10 including trade name, capacity, ingredient, manufacturer, production lot number and the like.

It should be noted that it is not requisite that the first controller 1A generates the donor-identification information BC as described in the first exemplary embodiment. Alternatively, the third controller 3A may generate the donor-identification information BC. In the alternative case, the generated donor-identification information BC is preferably transmitted to the first controller 1A and the second controller 2A through the network 4 in advance.

The label printer 3B receives the donor-identification information BC and the blood bag information having been stored in the third controller 3A and provides them on the blood bag 20.

In the first exemplary embodiment, the label printer 3B prints the donor-identification information BC or a barcode and the blood bag information to the same label sheet to produce the labels L1, and sticks the labels L1 on the blood bag 20. In the first exemplary embodiment, the blood bag 20 includes the bags 21, 22, 23, 24, 25, among which the first bag 21, the second bag 23 and the third bag 24 are stuck with the labels L1 printed with the donor-identification information BC.

In the label printer 3B, for instance, a rolled label sheet including unprinted labels arranged at predetermined intervals is set. While unrolling the label sheet, the label printer 3B prints the donor-identification information BC or a barcode and the blood bag information, feeds the labels L1, and sticks the labels L1 on each of the bags of the blood bag 20. The printable front sheet has a printable surface printed with the barcode and a surface opposite to the printable surface provided with the adhesive layer.

Preferably, the label printer 3B includes label printers as many as the bags of the blood bag 20 to be stuck with the labels L1. The plurality of label printers 3B may be arranged side by side correspondingly to the bags of the blood bag 20 arranged side by side so that the labels L1 printed with intended information and sized in accordance with the respective sizes of the corresponding bags are simultaneously manufactured and stuck on the corresponding bags. This results in an improvement in the manufacturing efficiency of the blood bag 20.

The label manufacturing device 3C is an identification information medium manufacturing device that receives the same donor-identification information BC as one received by the label printer 3B from the third controller 3A and manufactures the identification information labels 50 printed with the donor-identification information BC (barcode). In the first exemplary embodiment, the label manufacturing device 3C manufactures the six identification information labels 50 printed with a barcode and separably united to one another. In the label manufacturing device 3C, for instance, a rolled label sheet including unprinted labels that are separably united to one another and arranged at predetermined intervals is set in the same manner as in the label printer 3B. The label manufacturing device 3C prints the donor-identification information BC or a barcode to each of the identification information labels 50, and feeds the identification information labels 50. It should be noted that the label L2, which is manufactured by the label manufacturing device 3C, may be stuck on the cover 12 later or be immediately stuck on the cover 12 by the label printer 3B.

The third barcode reader 3D reads the donor-identification information BC or a barcode printed on the labels L1 and the identification information labels 50, and transmits the read information to the third controller 3A. Based on the information received from the third barcode reader 3D, the third controller 3A performs a predetermined process such as relating the received information to blood-collecting date and/or blood-collecting information.

As long as the third barcode reader 3D can read a barcode, the third barcode reader 3D is not specifically limited as well as the first barcode reader 1C.

The network 4 may be a wired network or a wireless network and may be a dedicated network or a public network. Specifically, networks such as a LAN and wide area networks such as domestic network and worldwide network are usable.

Next, a medical container management method according to the first exemplary embodiment will be described.

The medical container management method according to the first exemplary embodiment is configured to manage the blood bag 20 (the first medical container) and the blood collection tubes 30 (the second medical container), and preferably uses the above-described medical container management system S1. An exemplary arrangement of the management method using the medical container management system S1 will be described below. The first exemplary embodiment is, however, not intended to limit the invention.

Initially, the first controller 1A generates the donor-identification information BC in advance. The donor-identification information BC is transmitted to the second controller 2A and the third controller 3A through the network 4.

The third controller 3A then transmits the received donor-identification information BC to the label printer 3B in relation to the blood bag information inputted using the input device, while transmitting the same donor-identification information BC to the label manufacturing device 3C.

The label printer 3B then prints the donor-identification information BC or a barcode and the blood bag information on the same label sheet to produce the labels L1, and sticks the labels L1 on the blood bag 20. In the first exemplary embodiment, the blood bag 20 includes the bags 21, 22, 23, 24, 25, among which the first bag 21, the second bag 23 and the third bag 24 are stuck with the labels L1 printed with the donor-identification information BC and the blood bag information. The other bags, i.e., the blood test bag 22 and the fourth bag 25, are stuck with the labels L1 printed with only the blood bag information.

The label manufacturing device 3C manufactures the separably united identification information labels 50 printed with the donor-identification information BC or a barcode as many as objects to be stuck with the identification information labels 50 (e.g., the blood collection tubes 30 and medical record). The donor-identification information BC printed on the identification information labels 50 is the same in content as the donor-identification information BC printed on the labels L1 stuck on the blood bag 20.

Next, the medical container package 10 is manufactured by packing the blood bag 20 stuck with the labels L1 in the container body 11 of the medical container package 10 along with the identification information labels 50 and heat-sealing the cover 12 to close the opening 112 of the container body 11. It should be noted that the medical container package 10 is preferably subjected to sterilization before or after the cover 12 is heat-sealed. The cover 12 is stuck with the label L2 printed with the information about the medical container package 10 including trade name, capacity, ingredient, manufacturer, production lot number and the like and the donor-identification information BC in advance. It should be noted that the third controller 3A preferably stores the information printed on the label L2 in relation to the donor-identification information BC and the blood bag information in advance, while transmitting the information printed on the label L2 to the first controller 1A and the second controller 2A through the network 4.

The manufactured medical container package 10 is transported to a blood donation room and used there.

A blood-collecting person in the blood donation room, for instance, fills out a medical record with information about a donor, enters the information about the donor in the second controller 2A using the input device, and sticks one of the identification information labels 50 on the medical record. The second controller 2A relates the donor-identification information BC to be assigned to the donor to the information about the donor.

The blood-collecting person unseals the medical container package 10, takes out the blood bag 20 and the identification information labels 50, and sets the blood bag 20 in the blood collection device 2B to collect a predetermined amount of blood from the donor. The blood collection device 2B transmits the blood-collecting information including blood-collection conditions to the second controller 2A.

Using the second barcode reader 2C, the blood-collecting person reads at least one of the donor-identification information BC or a barcode printed on the label L1 on the blood bag 20, one printed on the label L2 on the medical container package 10, and one printed on the identification information labels 50 on the blood collection tubes 30. The second barcode reader 2C transmits the read information to the second controller 2A.

The blood-collecting person sets the united identification information labels 50 on the label mount 43 in the blood collection tube case 40, and distributes the blood contained in the blood test bag 22 among the five blood collection tubes 30, which are then closed with the plugs 31. Subsequently, the blood-collecting person arranges the blood collection tubes 30 side by side along the partitions 41 and packs the blood collection tubes 30 in the blood collection tube case 40. Incidentally, details of the above process for packing the blood collection tubes 30 in the blood collection tube case 40 are the same as described above.

The blood collection device 2B transmits the blood-collecting information regarding the blood collection to the second controller 2A. The second controller 2A stores the blood-collecting information in the storage in relation to the information about the donor and the donor-identification information BC, while transmitting the blood-collecting information to the first controller 1A through the network 4.

The blood bag 20 containing the blood is transported to the blood center along with blood collection tubes 30 arranged side by side in the blood collection tube case 40. Alternatively, the blood collection tubes 30 may be transported to the blood center after each being stuck with one of the united identification information labels 50, which are manually separated along a separable portion by the blood-collecting person.

A blood product maker in the blood center operates the blood product manufacturing equipment 1 B, for instance, to remove leukocytes from the blood contained in the first bag 21 through the blood filter 26 and second bag 23 and to separate red blood cells from blood plasma by centrifugation. The blood product manufacturing equipment 1B transmits the blood product manufacturing information regarding the manufacturing of the blood product to the first controller 1A. The first controller 1A stores the received blood product manufacturing information in the storage thereof in relation to the donor-identification information BC. Further, the stored blood product manufacturing information is preferably also related to various information transmitted from the second controller 2A and the third controller 3A.

The blood product maker reads the donor-identification information BC or a barcode printed on the labels L1 on the blood bag 20, and on the identification information labels 50 on the blood collection tubes 30 using the first barcode reader 1C. The first barcode reader 1C transmits the read information to the first controller 1A. Since the united identification information labels 50 are stuck on the blood collection tubes 30 arranged side by side in the blood collection tube case 40, the information can be collectively read from the identification information labels 50 using the first barcode reader 1C. A workload for reading the information can thus be reduced as compared to if the blood collection tubes 30 were taken one by one by hand to read the information. When the identification information labels 50 are separated, the blood collection tubes 30 may be taken one by one by hand to read the information.

After the donor-identification information BC on the blood collection tubes 30 is read, the united identification information labels 50 are separated from one another before taking the blood collection tubes 30 out of the blood collection tube case 40. The blood collection tubes 30 are thus each stuck with one of the identification information labels 50.

### Advantage(s) of First Exemplary Embodiment

The medical container package 10 includes the blood bag 20 stuck with the labels L1 printed with the donor-identification information BC. The blood-collecting person in the blood donation room thus does not need to stick a label printed with the donor-identification information BC to each bag each time when he/she collects blood. The blood-collecting person merely needs to stick the medical record and the identification information labels 50 on the blood collection tubes 30, among which the blood is partly distributed from the blood test bag 22.

The medical container package 10 can thus reduce a workload for sticking a label printed with the donor-identification information BC to the blood bag 20.

In the medical container management system S1, the label printer 3B receives the donor-identification information BC generated by the first controller 1A through the network 4 and the third controller 3A, and sticks the labels L1 printed with donor-identification information BC and the blood bag information on the blood bag(s) 20. Further, in the medical container management system S1, the label manufacturing device 3C receives the same information as the donor-identification information BC from the third controller 3A in the same manner as described above, and manufactures the identification information labels 50 printed with the donor-identification information BC.

The medical container management system S1 thus eliminates the need for independently preparing the identification information labels 50 as long as the identification information labels 50 are packed in the medical container package 10 along with the blood bag 20 having been provided with the donor-identification information BC. Further, the blood-collecting person does not need to stick a label printed with the donor-identification information BC to each of the bags of the blood bag 20 taken out of the medical container package 10 each time when he/she collects blood.

The medical container management system S1 can thus reduce a workload for sticking label printed with the donor-identification information BC to the medical containers.

In the method of sticking the identification information labels 50 to the blood collection tubes 30 according to the first exemplary embodiment, the separably united identification information labels 50 at least as many as the blood collection tubes 30 are placed on the label mount 43 with the adhesive surface of the adhesive layer 51 of each of the identification information labels 50 facing upward. Subsequently, the plurality of blood collection tubes 30 are arranged along the partitions 41 and packed in the blood collection tube case 40, thereby sticking the identification information labels 50 to the blood collection tubes 30. Alternatively, the united identification information labels 50 may be placed on the plurality of blood collection tubes 30 arranged along the partitions 41 with the adhesive surface of the adhesive layer 51 of each of the identification information labels 50 facing downward, thereby sticking the identification information labels 50 to the blood collection tubes 30.

In either of the above ways, the plurality of identification information labels 50, which are separably united, are positioned relative to the plurality of blood collection tubes 30 arranged side by side and stuck thereon as described above, so that the blood-collecting person does not need to stick each of the identification information labels 50 to corresponding one of the blood collection tubes 30 each time when he/she collects blood.

The method of sticking the identification information labels according to the first exemplary embodiment can thus reduce a workload for sticking the identification information labels 50 to the blood collection tubes 30.

In the medical container management method according to the first exemplary embodiment, the identification information labels 50 are stuck on the plurality of blood collection tubes 30 arranged side by side as in the same manner as in the method of sticking the identification information labels. The donor-identification information BC is then read from the identification information labels 50 stuck on the plurality of blood collection tubes 30 arranged side by side. The blood-collecting person thus does not need to stick each of the identification information labels 50 to corresponding one of the blood collection tubes 30 each time when he/she collects blood. Further, the blood-collecting person or the blood product maker can collectively read the donor-identification information BC printed on the identification information labels 50 on the blood collection tubes 30 instead of reading the information from the identification information labels 50 on the blood collection tubes 30 one by one.

The medical container management method according to the exemplary embodiment can thus reduce a workload for sticking the identification information labels 50 to the blood collection tubes 30 and a workload for managing the blood collection tubes 30.

The first exemplary embodiment reduces a workload for sticking the labels printed with the donor-identification information BC to the blood bag 20. This results in a reduction in the possibility of destroying the consistency between the blood-collecting person, the blood bag 20 and the blood collection tubes 30, and thus in an improvement in the accuracy of management of the collected blood.

Further, the donor-identification information BC is stored in the storage of the controller in relation to, for instance, the blood bag information, the information about the donor, the blood-collecting information and the blood product manufacturing information, which results in an improvement in the traceability of the blood product.

### Second Exemplary Embodiment

A second exemplary embodiment of the invention will be described below with reference to the attached drawings.

It should be noted that components identical to those of the first exemplary embodiment are attached with the like reference signs, and explanation thereof is simplified or omitted herein below.

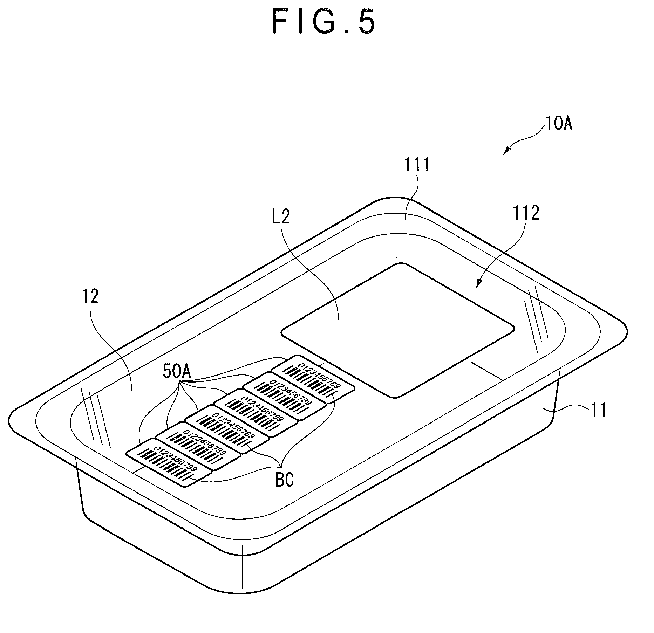

Fig. 5 is a perspective view of a medical container package 10A according to the second exemplary embodiment.

The medical container package 10A contains the blood bag 20 in the same manner as the medical container package 10 of the first exemplary embodiment, but is different from the medical container package 10 in that a surface of the cover 12 is stuck with the identification information labels 50A in addition to the label L2. The medical container package 10 of the first exemplary embodiment contains the identification information labels 50 in addition to the blood bag 20.

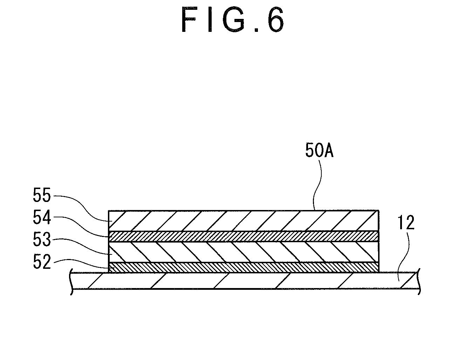

Fig. 6 is a schematic sectional view of the identification information labels 50A stuck on the surface of the cover 12. The identification information labels 50A, which are manufactured by the label manufacturing device 3C, each include a release liner, a first adhesive layer 52, an intermediate sheet 53, a second adhesive layer 54 and a printable front sheet 55, which are laminated in this sequence. The printable front sheet 55 is printed with the donor-identification information BC or a barcode. The identification information labels 50A are stuck on the blood collection tubes 30 by separating the second adhesive layer 54 from the intermediate sheet 53 and placing the second adhesive layer 54 on the blood collection tubes 30 with the printable front sheet 55 facing upward. It should be noted that Fig. 6 does not show the release liner, which has already been removed so that the identification information labels 50A are stuck on the cover 12. The printable front sheet 55 and the intermediate sheet 53 may be made of a material selected from among the materials listed for the labels L1. The first adhesive layer 52 and the second adhesive layer 54 may be made of a material selected from among the materials listed for the labels L1. The release liner may be made of a material selected from among the materials listed for the identification information labels 50. A surface of the intermediate sheet 53 to be in contact with the second adhesive layer 54 and a surface of the release liner to be in contact with the first adhesive layer 52 are subjected to a release treatment. Preferably, the surface of the release liner to be in contact with the first adhesive layer 52 is subjected to a light release treatment, whereas the surface of the intermediate sheet 53 opposite to a surface of the intermediate sheet 53 (i.e., the surface of the intermediate sheet 53 to be in contact with the first adhesive layer 52) is subjected to a heavy release treatment. In other words, a release force required to separate the intermediate sheet 53 from the second adhesive layer 54 is larger than a release force required to separate the first adhesive layer 52 from the release liner, so that the identification information labels 50A can be easily stuck on the cover 12.

In the second exemplary embodiment, the separably united identification information labels 50A may be removed from the surface of the cover 12 and stuck on the blood collection tubes 30 with the assistance of blood collection tube case 40. Alternatively, the identification information labels 50A may be separated and stuck one by one. However, the former way is preferable in terms of reduction in a workload. In sticking the identification information labels 50A to the blood collection tubes 30 in the former way, a portion of each of the separably united identification information labels 50A including the printable front sheet 55 should be removed from the cover 12. The second adhesive layer 54 is thus separated from the intermediate sheet 53. Subsequently, the identification information labels 50A removed from the cover 12 may be placed on the label mount 43 in the blood collection tube case 40 with an adhesive surface of the second adhesive layer 54 facing upward and a printable surface of the printable front sheet 55 facing downward as shown in Fig. 3B referred to above, or may alternatively be placed on the blood collection tubes 30 placed in the blood collection tube case 40 with the adhesive surface of the second adhesive layer 54 facing downward and the printable surface of the printable front sheet 55 facing upward as shown in Fig. 3D referred to above.

The label printer 3B may be used in place of the label manufacturing device 3C so that the identification information labels 50A are stuck on the surface of the cover 12 immediately when manufactured, which results in preventing the identification information labels 50A from failing to be being packed in the medical container package 10A. Further, this arrangement does not require the release liner to be wasted, which results in a reduction of wastes.

The medical container package 10A of the second exemplary embodiment provides effects similar to those of the first exemplary embodiment. The medical container package 10A of the second exemplary embodiment may be used in combination with the blood collection tube case 40 of the first exemplary embodiment to implement the medical container management system, method of sticking identification information labels and medical container management method of the first exemplary embodiment and provide similar effects.

### Third Exemplary Embodiment

A third exemplary embodiment of the invention will be described below with reference to the attached drawings.

It should be noted that components identical to those of the first and second exemplary embodiments are attached with the like reference signs, and explanation thereof is simplified or omitted herein below.

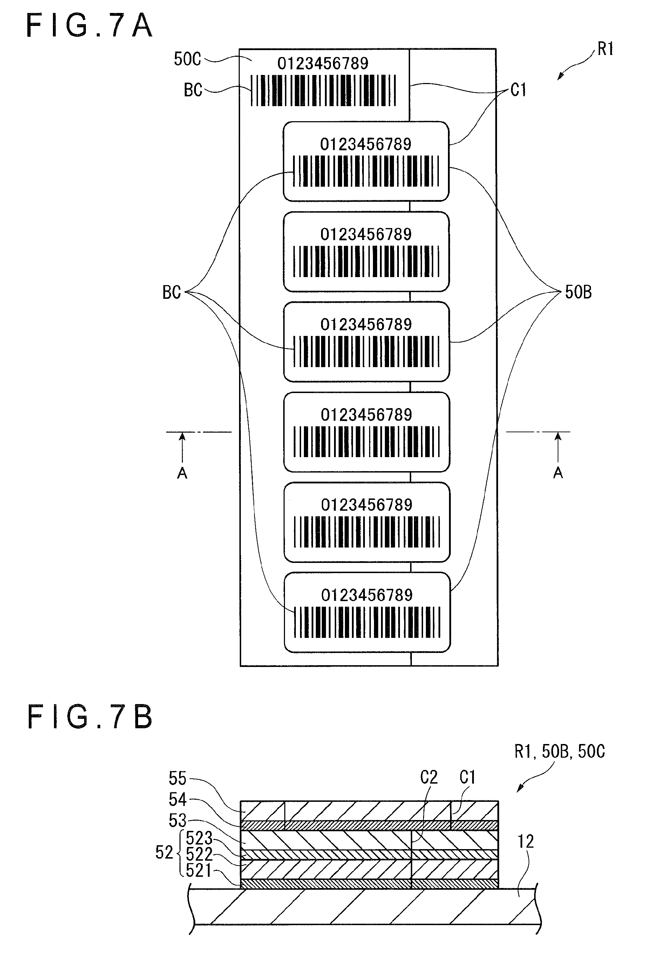

Fig. 7A is a plan view of a first united label body R1 including united identification information labels 50B to be stuck on the cover 12 of a medical container package according to the third exemplary embodiment, and Fig. 7B is a schematic sectional view of the first united label body R1 stuck on a surface the cover 12 as viewed in a direction of arrows A-A.

In the third exemplary embodiment, the first united label body R1 includes the six identification information labels 50B that are separably united to one another, and one identification information label 50C with a length longer than a total length of the identification information labels 50B. The size of the container body 11 is not specifically limited as long as it can accommodate at least the blood bag 20.

The first united label body R1 is manufactured by the label manufacturing device 3C through a process including printing and punching. The first united label body R1 includes the release liner, which has already been removed and is not shown in Figs. 7A and 7B, the first adhesive layer 52, the intermediate sheet 53, the second adhesive layer 54 and the printable front sheet 55, which are laminated in this sequence. The first adhesive layer 52 includes a cover-side adhesive layer 521, a core 522 and a peelable adhesion layer 523, which are laminated on the cover 12 in this sequence.

The first united label body R1 is provided with a cut C1 formed at a position determined by an area where the identification information labels 50B and the identification information label 50C are present. The cut C1 is formed not to overlap the identification information labels 50B and a portion of the identification information label 50C printed with the donor-identification information BC, and has a depth from the printable front sheet 55 to the second adhesive layer 54. The cut C1 is formed by punching the first united label body R1 from a side corresponding to the printable front sheet 55.

The first united label body R1 is also provided with a cut C2. The cut C2 is formed by punching the first united label body R1 from a side corresponding to the release liner to the intermediate sheet 53. The cut C2 has a depth from the release liner to the intermediate sheet 53. It should be noted that Fig. 7B does not show the release liner, which has already been removed so that the cover-side adhesive layer 521 of the first adhesive layer 52 are stuck on the cover 12.

It should be noted that the punching for forming the cut C1 and the cut C2 is a process intended to form a cut having not a depth sufficient to penetrate through the first united label body R1 in a thickness direction thereof, but a depth sufficient to reach a predetermined position in the thickness direction.

The core 522 includes a base made of any one of: synthetic resin films such as acrylic resin, polycarbonate resin, polyethylene resin, polypropylene resin, polyvinyl chloride resin, acrylonitrile·butadiene·styrene resin, polyester resin (e.g., polyethylene terephthalate) and polyimide resin, and paper materials such as wood-free paper, impregnated paper, glassine paper, coated paper and non-woven cloth. The thickness of the core 522 is usually selected in a range from 5 µm to 300 µm, more preferably from 20 µm to 250 µm. The above range is determined in view of the fact that the core 522 with a thickness of less than 5 µm is poor in mechanical strength and thus is likely to deform depending on external pressure conditions. Further, the core 522 with a thickness of more than 300 µm may be difficult to, for instance, roll or cut.

The peelable adhesion layer 523 is not specifically limited as long as it can peelably adhere to the core 522 and the intermediate sheet 53, but is preferably a thermoplastic resin layer. Examples of the thermoplastic resin include polyethylene, polypropylene, polymethyl and pentene. These thermoplastic resins may be used alone or a mixture of two or more thereof may be used. The thickness of the peelable adhesion layer 523 is not specifically limited, but may be in a range from 3 µm to 70 µm, preferably from 5 µm to 50 µm. The above range is determined in view of the fact that the peelable adhesion layer 523 with a thickness of less than 3 µm may fail to exhibit an adhesive force. Further, the peelable adhesion layer 523 with a thickness of more than 70 µm may make the identification information labels 50 excessively thick as a whole.

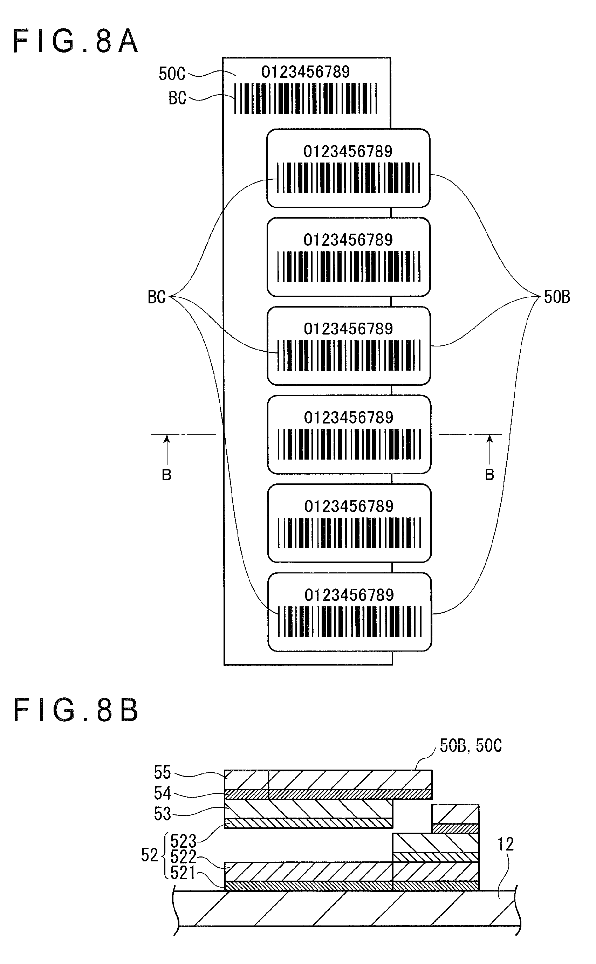

Fig. 8A is a plan view showing a part of a process for sticking each of the identification information labels 50B to corresponding one of the blood collection tubes 30, and Fig. 8B is a schematic sectional view showing the same as viewed in a direction of arrows B-B.

When the peelable adhesion layer 523 is separated from the core 522 along an interface therebetween to remove the portion of the identification information label 50C printed with the donor-identification information BC from the cover 12 along the cut C1, a remaining portion of the identification information label 50C, which is unprinted with the donor-identification information BC, is left on the cover 12 as shown in Fig. 8A. The identification information labels 50B project from the intermediate sheet 53 of the removed identification information label 50C. In the third exemplary embodiment, each of the identification information labels 50B may be stuck on corresponding one of the blood collection tubes 30 by pinching the projection of each of the identification information labels 50B and separating each of the identification information labels 50B from the first united label body R1. The identification information labels 50B can thus be easily separated from the first united label body R1 and stuck with an improved efficiency.

It should be noted that the medical container package of the third exemplary embodiment provides effects similar to those of the first exemplary embodiment. The medical container package of the third exemplary embodiment may be used to implement the medical container management system and medical container management method of the first exemplary embodiment and provide similar effects.

### Fourth Exemplary Embodiment

A fourth exemplary embodiment of the invention will be described below with reference to the attached drawings.

It should be noted that components identical to those described in the first to third exemplary embodiments are attached with the like reference signs, and explanation thereof is simplified or omitted herein below.

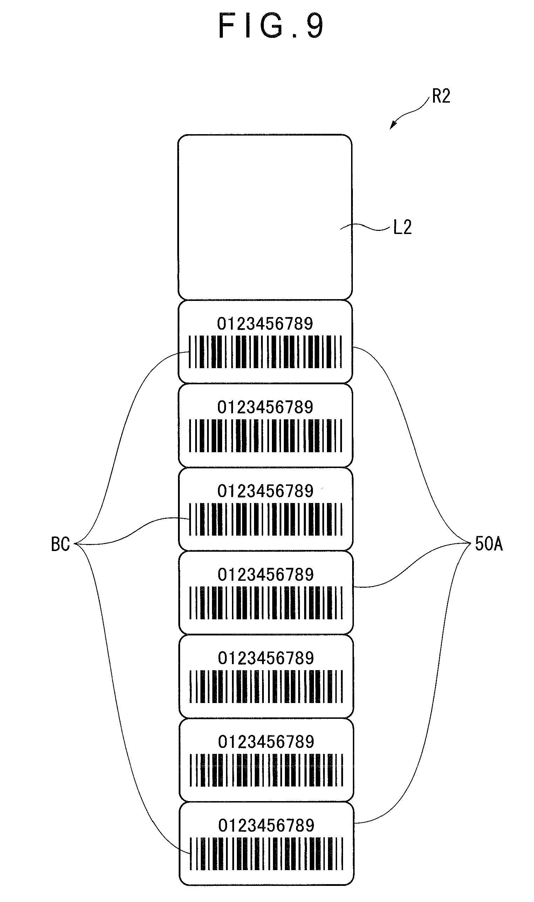

Fig. 9 is a plan view of a second united label body R2 including the identification information labels 50A and the label L2 that are to be stuck on the cover surface of a medical container package according to the fourth exemplary embodiment, the identification information labels 50A and the label L2 being separably united.

The second united label body R2, which has a cross-sectional arrangement identical to one shown in Fig. 6, is manufactured by the label manufacturing device 3C. The second united label body R2 includes the release liner, the first adhesive layer 52 that is to be adhered to the cover 12, the intermediate sheet 53, the second adhesive layer 54 and the printable front sheet 55, which are laminated in this sequence. In the fourth exemplary embodiment, the label printer 3B may be used in place of the label manufacturing device 3C so that the second united label body R2 is stuck on the surface of the cover 12 immediately when manufactured. The label L2 and the identification information labels 50A can thus be stuck on the cover 12 of the medical container package of the fourth exemplary embodiment through the same process, which results in an improved efficiency in manufacturing the medical container package. It should be noted that the identification information labels 50A of the fourth exemplary embodiment may be configured similarly to those of the first united label body R1 of the third exemplary embodiment.

The medical container package of the fourth exemplary embodiment provides effects similar to those of the first exemplary embodiment. The medical container package of the fourth exemplary embodiment may be used to implement the medical container management system and medical container management method of the first exemplary embodiment and provide similar effects.

### Fifth Exemplary Embodiment

Next, a fifth exemplary embodiment of the invention will be described.

It should be noted that components identical to those described in the first to fourth exemplary embodiments are attached with the like reference signs, and explanation thereof is simplified or omitted herein below.

The medical container package according to the fifth exemplary embodiment includes the blood collection tubes 30 stuck with the separably united identification information labels 50A; and the blood bag 20 stuck with the label L1. In other words, the blood collection tubes 30 each stuck with one of the identification information labels 50A and the blood bag 20 stuck with the label L1 are packed in the medical container package according to the fifth exemplary embodiment.

Therefore, a blood-collecting person in a blood donation room thus does not need to stick a label printed with the donor-identification information BC to each of the blood bag 20 and the blood collection tubes 30 each time when he/she collects blood. This results in a reduction in a workload. Consequently, the blood-collecting person thus only has to distribute blood contained in the blood test bag 22 among the labeled blood collection tubes 30 also packed in the medical container package.

The medical container package of the fifth exemplary embodiment can thus eliminate the need for sticking a label printed with the donor-identification information BC to each medical container (e.g., the blood bag 20 and the blood collection tubes 30).

The medical container package of the fifth exemplary embodiment can be used to implement the medical container management system and the medical container management method of the first exemplary embodiment and provide similar effects.

Especially, since the fifth exemplary embodiment eliminates the need for sticking a label printed with the donor-identification information BC to each of the blood bag 20 and the blood collection tubes 30, an operation error such as forgetting to stick a label and sticking a wrong label can be avoided. This results in a reduction in the possibility of destroying the consistency between the blood-collecting person, the blood bag 20 and the blood collection tubes 30. The collected blood can thus be managed with improved accuracy.

### Modifications of Exemplary Embodiments

Incidentally, it should be understood that the scope of the invention is not limited to the above-described exemplary embodiment(s) but includes modifications and improvements compatible with the invention.

The medical container is, for instance, in the shape of a bag according to the exemplary embodiments, but may be in a different shape without departing from the scope of the invention. For instance, the medical container may be in the shape of a bottle or a pouch.

The blood bag (the medical container) is stuck with the a label according to the exemplary embodiments, but may be stuck with any other information media without departing from the scope of the invention.

The label L1 stuck on each of the first bag 21, the second bag 23 and the third bag 24 is printed with the donor-identification information BC according to the exemplary embodiments, which is not requisite for the blood bag (the medical container) of the invention. The medical container is merely required to have the donor-identification information, and the identification information medium stuck on the medical container may alternatively be a radio frequency identification (RFID) tag. A label printed with the donor-identification information BC as in the exemplary embodiments is preferably subjected to a laminating process to prevent the printed information from disappearing. In contrast, the RFID tag usable in place of the label eliminates the need for the laminating process, which results in an improvement in the manufacturing efficiency and the preservation stability of the identification information. Further, the RFID tag is capable of electromagnetic contact or non-contact reading/writing of information using a reader/writer, which enables reading of a large amount of information with high accuracy as compared with optical reading of printed information such as a barcode. The RFID tag is also capable of addition of information in each process, so that the history of each process can be traced using only the RFID tag without the network. However, the RFID tag is costly as compared with the label, so that the RFID tag or the label may be selectively used in view of a balance between costs and required accuracy. The donor identification information may be printed directly on the blood bag (the medical container) according to another exemplary embodiment. A method of directly printing the information is not specifically limited as long as the information can be printed. For instance, thermal transfer printing, laser, laser beam printing or ink-jet printing may be employed.

The donor-identification information BC may be read by optical character recognition using an optical character reader (OCR) instead of barcode recognition.

In the exemplary embodiments, the blood collection tubes 30 (the collection container) are placed, for instance, on the case bottom 42 of the box-shaped blood collection tube case 40 (the collection container storing case), which is not requisite for the invention. Alternatively, a blood collection tube case where the blood collection tubes 30 are to be arranged in an upright position or a blood collection tube case where the blood collection tubes 30 are to be arranged in a tilted position may be used.

In the exemplary embodiments, the blood collection tube case 40 is, for instance, a box in the shape of a rectangular parallelepiped and opening upward, which is not requisite for the invention. For instance, the blood collection tube case 40 may include the partitions 41 and the case bottom 42 but not include a peripheral wall. It is not requisite that the partitions 41 be vertically disposed on the case bottom 42. Alternatively, the case bottom 42 may be uneven due to the presence of partitions in the form of grooves extending in the longitudinal directions of the blood collection tubes 30. Specifically, the grooves as many as the blood collection tubes 30 to be placed may be formed side by side so that the longitudinal directions of the plurality of blood collection tubes 30 placed along the grooves are oriented in the same direction. The identification information labels 50 may be stuck on the thus-arranged blood collection tubes 30.

The blood collection tube case 40 may be packed in the medical container package along with tools for collecting blood including the blood bag 20 and the blood collection tubes 30, thereby improving the work efficiency of a blood-collecting person (a person who collects a medical fluid) in the blood donation room.

In the exemplary embodiments, the plurality of blood collection tubes 30 are, for instance, collectively stuck with the identification information labels 50 with the assistance of the blood collection tube case 40, but may alternatively be individually stuck without using the blood collection tube case 40. Further, the blood collection tubes 30 may be taken one by one to read the barcode thereon using a barcode reader.

### INDUSTRIAL APPLICABILITY

The present invention is applicable to a medical container package and a medical container management system.

### EXPLANATION OF CODE(S)

- 10, 10A: medical container package
- 20: blood bag (medical container)
- 30, 30a: blood collection tube (collection container, medical container)
- 50, 50A, 50B, 50C: identification information label
- S1: medical container management system
- 1A: first controller (controller)
- 2A: second controller (controller)
- 3A: third controller (controller)
- 3B: label printer (identification information providing device)

## Claims

1. A medical container package comprising:
an identification information medium indicating donor-identification information that is to be assigned to each donor of a medical fluid; and
a medical container configured to contain the medical fluid, the medical container having been provided with donor-identification information that is the same in content as the donor-identification information indicated by the identification information medium.

2. A medical container management method comprising managing a medical container configured to contain a medical fluid on a basis of each donor of the medical fluid using a medical container package, the medical container package comprising:
the medical container having been provided with donor-identification information that is to be assigned to the each donor, the donor-identification information being generated in advance; and
an identification information medium indicating donor-identification information that is the same in content as the donor-identification information provided on the medical container.

3. A medical container management method comprising:
providing donor-identification information that is to be assigned to each donor of a medical fluid to a first medical container in advance;
preparing an identification information medium printed with donor-identification information that is the same in content as the donor-identification information provided on the first medical container;
packing the first medical container in a medical container package;
providing the identification information medium to the medical container package;
reading the donor-identification information provided on the first medical container;
storing the read donor-identification information in relation to information about the each donor; and
sticking the identification information medium to a second medical container.
